**Europäisches Patentamt**

**European Patent Office**

**Office européen des brevets**

(19)

(11) Numéro de publication : **0 370 913 B1**

(12)

# FASCICULE DE BREVET EUROPEEN

(45) Date de publication du fascicule du brevet :
**23.12.92 Bulletin 92/52**

(51) Int. Cl.⁵ : **A23P 1/08,** A61K 9/48

(21) Numéro de dépôt : **89403241.6**

(22) Date de dépôt : **23.11.89**

(54) **Film comestible à base de farine de blé à basse ou moyenne teneur en protéines, son procédé de préparation et son utilisation comme emballage.**

(30) Priorité : **24.11.88 FR 8815336**

(43) Date de publication de la demande :
**30.05.90 Bulletin 90/22**

(45) Mention de la délivrance du brevet :
**23.12.92 Bulletin 92/52**

(84) Etats contractants désignés :
**AT BE CH DE ES FR GB GR IT LI LU NL SE**

(56) Documents cités :
**DE-A- 2 708 513**
**FR-A- 2 087 185**
**US-A- 2 846 353**
**WORLD PATENT INDEX, no. 85-321813/51,**
**Derwent Publications Ltd, London, GB**

(73) Titulaire : **LES LABORATOIRES DE L'ECOLE NATIONALE SUPERIEURE DE MEUNERIE ET DES INDUSTRIES CEREALIERES**
**66 rue de la Boétie**
**F-75008 Paris (FR)**

(72) Inventeur : **Mazerand, Claire**
**82, rue de Sèvres**
**F-75007 Paris (FR)**
Inventeur : **Draproon, Roger**
**4, rue des Mariniers**
**F-75014 Paris (FR)**
Inventeur : **Lamballais, Michel**
**4, Allée des Airs**
**F-78720 Saint Forget les Sablons (FR)**

(74) Mandataire : **Lepeudry-Gautherat, Thérèse et al**
**ARMENGAUD JEUNE CABINET LEPEUDRY**
**52, avenue Daumesnil**
**F-75012 Paris (FR)**

## Description

La présente invention concerne un film comestible destiné à l'emballage, l'enrobage ou la séparation de denrées, et son procédé de préparation à partir d'un matériau constitué principalement de farine de blé à basse ou moyenne teneur en protéines additionnée d'un ou plusieurs agents plastifiants.

L'utilisation d'emballages ou d'enrobages (c'est-à-dire de substances appliquées directement sur l'aliment) se pratique de manière empirique depuis fort longtemps : en Europe, l'utilisation de graisse pour protéger la viande se pratique depuis le 16ème siècle et le pelliculage des fruits à la cire se pratique en Chine depuis le 12ème siècle.

Depuis une quarantaine d'années, de nombreux travaux ont été réalisés pour mettre au point des films comestibles destinés à la conservation de divers aliments frais, séchés, cuisinés ou congelés. Divers matériaux glucidiques, protéiques, lipidiques, simples ou hétérogènes, ont été utilisés. On citera plus particulièrement des films protéiques dérivés de zéine, surtout étudiés aux U.S.A. et des films de lipoprotéines dérivés du lait de soja, surtout utilisés en Asie.

On trouvera une revue des diverses formulations et technologies connues dans les publications de Guilbert (dans "Food packaging and préservation" M. Mathlouthi. ed. Elsevier N.Y. 1986 pp 371-394) et de Kester et Fennema (Food Technol. 40, 1986, 47-59).

Diverses substances peuvent être ajoutées pendant la fabrication des films pour améliorer leurs propriétés mécaniques, protectrices ou sensorielles, On distingue en particulier les agents plastifiants qui modifient l'organisation et les forces intermoléculaires ainsi que la structure tridimensionnelle du matériau filmogène et en augmentent la cohésion et la capacité d'élongation.

La nature du plastifiant est choisie en fonction de la nature du film ; les plus couramment utilisés sont les oligosaccharides, les polyols, les lipides et leurs dérivés (acides gras, monoglycérides et phospholipides...).

Pour être qualifié de comestible, un film d'emballage ou d'enrobage doit pouvoir faire partie intégrante de l'aliment ou de la substance qu'il protège; il doit donc avoir de bonnes propriétés sensorielles et être soluble ou dispersible dans l'eau ou dans la bouche.

De nombreux films d'emballage ou d'enrobage déjà connus présentent le désavantage, soit d'être insolubles ou difficilement dispersibles dans la bouche, soit de présenter une consistance désagréable, par exemple trop collante, soit de présenter des caractéristiques physico-chimiques et mécaniques défectueuses. Il serait donc bénéfique de disposer d'une méthode simple de production d'un film comestible, dérivé d'un matériel biologique peu coûteux, dont la composition pourrait être adaptée à diverses applications spécifiques et qui présenterait des qualités mécaniques et sensorielles améliorées.

La Demanderesse a ainsi mis au point un film comestible pouvant être préparé par un procédé simple à partir d'un mélange de farine de blé à basse ou moyenne teneur en protéines et d'un agent plastifiant tel que le glycérol; ledit procédé comprend un chauffage, une homogénéisation, un étalement et un séchage dans des conditions déterminées.

Le film obtenu par la mise en oeuvre de ce procédé, est comestible, totalement biodégradable et utilisable dans l'industrie alimentaire, pharmaceutique ou toute industrie où son caractère comestible et/ou biodégradable représente un avantage.

Le matériau de départ est une farine de blé tendre dont la teneur en protéines est comprise entre 3 et 15%.

Selon un mode de réalisation de l'invention, le matériau de départ est une farine à basse teneur en protéines, obtenue par exemple par turboséparation d'un broyat de farine de blé tendre. Cette méthode permet d'obtenir des fractions riches en gluten destinées en particulier à la panification et des fractions pauvres contenant de 3 à 8 % de protéines. Ces farines pauvres sont surtout utilisées en biscuiterie : La Demanderesse a trouvé ainsi de nouvelles applications de ce type de farine dont les débouchés sont actuellement assez limités.

L'invention concerne donc en particulier un procédé qui permet de préparer un film comestible en utilisant de la farine de blé tendre à basse ou moyenne teneur en protéines, plus particulièrement moins de 15 % en poids sec et, comme agent plastifiant, du glycérol ou d'autres polyols alimentaires, des glycérides, du sirop de glucose ou de fructose, des lipides ou des phospholipides, seuls ou en mélange, à une concentration comprise entre 1 et 30 % par rapport à la farine.

Selon un mode de réalisation préféré de l'invention, la concentration de farine mise en dispersion dans l'eau est d'environ 50 g/l et la concentration de glycérol comprise entre 15 et 30 %.

Selon un autre mode de réalisation de l'invention, le mélange farine-glycérol-eau est additionné d'huile comestible à une concentration inférieure ou égale à 10 %.

Selon un autre mode de réalisation de l'invention, on ajoute à la suspension aqueuse de farine et de plastifiant(s) du gluten ou un constituant du gluten, plus particulièrement les gliadines, à une concentration qui ne dépasse pas 30 %.

Le procédé selon l'invention comprend ensuite un chauffage au bain-marie, préférentiellement à une température voisine de 90 à 100°C et pendant un temps court, de l'ordre de 6 à 15 minutes. On obtient, par ce procédé, une suspension visqueuse qui est ensuite homogénéisée puis étalée en couche uniforme sur un support anti-adhésif, par exemple recouvert de polytétrafluoroéthylène ou de nylon, et mise à sécher jusqu'à obtention d'un film qui se détache aisément du support.

L'invention concerne donc également un film constitué de farine à basse ou moyenne teneur en protéines, de glycérol et éventuellement de lipides, qui est comestible et destiné à l'emballage, l'enrobage ou la séparation de denrées, selon des procédés applicables dans les industries alimentaire, pharmaceutique et autres.

Ce film peut être utilisé avantageusement dans l'industrie alimentaire pour limiter la dessiccation ou l'oxydation d'aliments frais ou congelés ou pour limiter la pénétration d'huile dans des aliments destinés à être frits. Ce film peut être utilisé pour séparer différentes couches d'un aliment composé, frais ou congelé, par exemple pour séparer la pâte et la garniture d'aliments de type pizza ou pour séparer les couches de différentes natures et textures dans les pâtisseries et biscuits hétérogènes.

Ce film peut aussi avantageusement être utilisé pour emballer des aliments, des condiments, des ingrédients quelconques, prédosés et destinés à être solubilisés rapidement, ou encore pour emballer le même type d'ingrédients mis en conserve ou surgelés et destinés à être réchauffés, ébouillantés ou intégrés dans une préparation. Ceci n'entraîne pas nécessairement la suppression d'un emballage ordinaire mais permet d'utiliser pour celui-ci des matériaux moins performants et moins coûteux.

De manière similaire, ce film peut être utilisé dans l'industrie pharmaceutique pour encapsuler divers produits, en poudre en particulier, et permettre leur dissolution et leur assimilation rapides.

Ce film peut également être utilisé dans tout autre type d'industrie où un emballage biodégradable ou comestible représente un avantage. Un tel emballage est particulièrement approprié pour les produits et les jouets destinés aux jeunes enfants.

Différents additifs peuvent être incorporés en cours de fabrication du film en fonction de l'usage spécifique auquel il sera destiné et ceci, plus particulièrement, en vue d'obtenir un effet localisé à la surface de la substance ou de l'aliment emballé ou enrobé. Ainsi on pourra y incorporer des agents antimicrobiens ou antioxydants autorisés dans l'industrie alimentaire, des colorants naturels ou synthétiques autorisés, des agents de sapidité (sel, sucres, arômes).

Les exemples suivants illustrent l'invention sans toutefois en limiter la portée.

EXEMPLE 1

La farine de blé tendre à basse teneur en protéines, obtenue par turboséparation, contient 7 %, en poids sec, de protéines. Cette farine présente les caractéristiques rhéologiques (mesurées à l'alvéographe de Chopin) suivantes : P = 41 ; G = 17,3 ; W = 77 ; P/L = 0,68.

On met en suspension 1 g de farine à basse teneur en protéines dans 20 ml d'eau; on ajoute 25 % de glycérol comme plastifiant.

La suspension est chauffée à 90°C au bain-marie pendant 6 minutes, sous agitation manuelle et on observe une gélification de la suspension. On prélève 10 ml de suspension pour l'étaler de manière uniforme sur un disque recouvert de polytétrafluoroéthylène, d'un diamètre de 12 cm. On laisse sécher à température ambiante, sous un ventilateur, pendant 75 minutes. Dans ces conditions, un film uniforme d'une épaisseur d'environ 0,1 mm est formé. Il se détache facilement et ses propriétés peuvent être évaluées : il est

- transparent (éventuellement légèrement opalescent mais pas translucide),
- souple et résistant au pliage et aux chocs,
- thermosoudable,
- dispersible dans la bouche et dans l'eau chaude et sans goût dominant.

EXEMPLE 2

On met en suspension 1g de farine de blé contenant 11 % en poids sec de protéines dans 20 ml d'eau ; on ajoute 200 mg de glycérol et 50 mg d'huile comestible, comme plastifiants, et 20 mg de chlorure de sodium ; le pH est ajusté à 4.

La suspension est chauffée au bain-marie à 100°C pendant 15 minutes, sous agitation mécanique. On observe une augmentation de viscosité de la suspension qui est alors homogénéisée. On prélève 10 ml de suspension pour l'étaler de manière uniforme sur un disque recouvert de nylon d'un diamètre de 12 cm.

On laisse sécher à une température voisine de 45°C sous un ventilateur, jusqu'à obtention d'un film qui se détache facilement.

EXEMPLE 3

Le procédé mis en oeuvre dans les exemples 1 et 2 peut être précédé d'une étape de prégélatinisation de la farine, par cuisson-extrusion.

**Revendications**

1. Film comestible destiné à l'emballage, l'enrobage ou la séparation de denrées, caractérisé en ce qu'il est obtenu à partir d'une farine de blé à basse ou moyenne teneur en protéines comprise entre 3 et 15 %, additionnée d'un agent plastifiant à une concentration de 1 à 30 % du poids sec de farine.

2. Film comestible selon la revendication 1, caractérisé en ce qu'il contient un ou plusieurs agents antioxydants autorisés dans l'industrie alimentaire.

3. Film comestible selon la revendication 1 ou 2, caractérisé en ce qu'il contient un agent de sapidité.

4. Film comestible selon l'une quelconque des revendications 1 à 3, caractérisé en ce qu'il contient un ou plusieurs agents antimicrobiens autorisés dans l'industrie alimentaire

5. Film comestible selon l'une quelconque des revendications 1 à 4, caractérisé en ce qu'il contient un ou plusieurs colorants autorisés dans l'industrie alimentaire et/ou pharmaceutique.

6. Procédé de préparation de films selon l'une quelconque des revendications 1 à 5, caractérisé en ce qu'on soumet la farine à un traitement comprenant une mise en suspension dans l'eau en présence d'agents plastifiants, un chauffage, une homogénéisation, un étalement sur un support anti-adhésif et un séchage.

7. Procédé selon la revendication 6, caractérisé en ce que la farine contient moins de 15 % en poids sec de protéines.

8. Procédé selon la revendication 6, caractérisé en ce que la farine à basse teneur en protéines contient moins de 8 % en poids sec de protéines.

9. Procédé selon l'une quelconque des revendications 6 à 8, caractérisé en ce que la farine est mise en suspension dans l'eau à une concentration d'environ 50 g/l, à un pH de 4 à 10.

10. Procédé selon l'une quelconque des revendications 6 à 9, caractérisé en ce qu'on ajoute à la suspension aqueuse de farine, du gluten ou un de ses constituants comme les gliadines, à une concentration qui ne dépasse pas 30 % par rapport à la farine.

11. Procédé selon l'une quelconque des revendications 6 à 10, caractérisé en ce qu'on ajoute à la suspension aqueuse de farine, un agent plastifiant choisi parmi le glycérol, les polyols, les glycérides, les phospholipides, les lipides, seul ou en mélange, à une concentration comprise entre 1 et 30 % par rapport à la farine.

12. Procédé selon la revendication 11, caractérisé en ce que le plastifiant est le glycérol.

13. Procédé selon la revendication 11 ou 12, caractérisé en ce que le plastifiant est une huile comestible.

14. Procédé selon l'une quelconque des revendications 6 à 13, caractérisé en ce que la suspension aqueuse de farine et d'agents plastifiants est gélifiée par chauffage au bain-marie.

15. Procédé selon la revendication 14, caractérisé en ce que la suspension est chauffée à une température de 90 à 100°C pendant 6 à 15 minutes.

16. Procédé selon l'une quelconque des revendications 6 à 15, caractérisé en ce que la suspension gélifiée est homogénéisée puis étalée en couche uniforme sur un support anti-adhésif et mise à sécher jusqu'à obtention d'un film.

**Claims**

1. An edible film to be used for wrapping, coating or separating food, characterized in that it is obtained from a wheat flour having a low or medium protein content between 3 and 15 % with addition of a plasticizer at a concentration of 1 to 30 % of the dry weight of the flour.

2. An edible film according to claim 1, characterized in that it contains one or several antioxidants permitted in the foodstuffs industry.

3. An edible film according to claim 1 or 2, characterized in that it contains a flavouring agent.

4. An edible film according to any of claims 1 to 3, characterized in that it contains one or several antimicrobial agents permitted in the foodstuffs industry.

5. An edible film according to any of claims 1 to 4, characterized in that it contains one or several colouring agents permitted in the foodstuffs and/or pharmaceutical industry.

6. A process for preparing films according to any of claims 1 to 5, characterized in that the flour is subjected to a treatment comprising suspension in water in presence of plasticizers, heating, homogenizing, spreading onto an anti-adhesive support and drying.

7. A process according to claim 6, characterized in that the flour contains less than 15 % of proteins, in relation to its dry weight.

8. A process according to claim 6, characterized in that the flour of low protein content contains less than 8 % of proteins, in relation to its dry weight.

9. A process according to any of claims 6 to 8, characterized in that the flour is suspended in water at a concentration of about 50 g/l at a pH of 4 to 10.

10. A process according to any of claims 6 to 9, characterized in that gluten or one of its constituents, such as gliadins, are added to the aqueous suspension of flour at a concentration not exceeding 30 % in relation to the flour.

11. A process according to any of claims 6 to 10, characterized in that a plasticizer selected from glycerol, polyols, glycerides, phospholipids and lipids, alone or in a mixture, is added to the aqueous suspension of flour at a concentration between 1 and 30 % in relation to the flour.

12. A process according to claim 11, characterized in that the plasticizer is glycerol.

13. A process according to claim 11 or 12, characterized in that the plasticizer is an edible oil.

14. A process according to any of claims 6 to 13, characterized in that the aqueous suspension of flour and plasticizers is gelled by heating in a water bath.

15. A process according to claim 14, characterized in that the suspension is heated at a temperature of 90 to 100 °C for 6 to 15 minutes.

16. A process according to any of claims 6 to 15, characterized in that the gelled suspension is homogenized and then spread as a uniform layer on an anti-adhesive support and allowed to dry until a film is formed.

**Patentansprüche**

1. Eßbare Folie bestimmt zur Verpackung, Umhüllung oder Trennung von Nahrungsmitteln, dadurch gekennzeichnet, daß sie aus einem Getreidemehl mit niedrigem oder mittlerem Proteingehalt im Bereich zwischen 3 und 15 % gewonnen ist unter Hinzufügung eines Plastifizierungsmittels mit bis zu einer Konzentration von 1 bis 30 % des Trockengewichtes des Mehles.

2. Eßbare Folie nach Anspruch 1, dadurch gekennzeichnet, daß sie ein oder mehrere in der Lebensmittelindustrie zugelassene oxidationsverhindernde Mittel enthält.

3. Eßbare Folie nach Anspruch 1 oder 2, dadurch gekennzeichnet, daß sie ein Geschmacksmittel enthält.

4. Eßbare Folie nach einem der Ansprüche 1 bis 3, dadurch gekennzeichnet, daß sie ein oder mehrere in der Lebensmittelindustrie zugelassene antimikrobische Mittel enthält.

5. Eßbare Folie nach einem der Ansprüche 1 bis 4, dadurch gekennzeichnet, daß sie ein oder mehrere in der Lebensmittelindustrie und/oder der pharmazeutischen Industrie zugelassene Färbemittel enthält.

6. Verfahren zur Herstellung von Folien nach einem der Ansprüche 1 bis 5, dadurch gekennzeichnet, daß man das Mehl einer Behandlung unterzieht enthaltend eine Suspensionierung in Wasser in Gegenwart von Plastifizierungsmitteln, eine Erhitzung, eine Homogenisierung, eine Ausbreitung auf einem nicht klebenden Träger und eine Trocknung.

7. Verfahren nach Anspruch 6, dadurch gekennzeichnet, daß das Mehl einen Trockengewichtsanteil an Proteinen von weniger als 15 % aufweist.

8. Verfahren nach Anspruch 6, dadurch gekennzeichnet, daß das Mehl mit niedrigem Proteingehalt weniger als einen Trockengewichtsanteil von 8 % an Proteinen aufweist.

9. Verfahren nach einem der Ansprüche 6 bis 8, dadurch gekennzeichnet, daß das Mehl in Wasser in Suspension gebracht ist mit einer Konzentration von ungefähr 50 g/l mit einem pH-Wert von 4 bis 10.

10. Verfahren nach einem der Ansprüche 6 bis 9, dadurch gekennzeichnet, daß man zu der wässrigen Mehlsuspension Gluten oder einen seiner Bestandteile wie die Gliadine mit einer Konzentration hinzufügt, die unter Bezug auf das Mehl 30 % nicht übersteigt.

11. Verfahren nach einem der Ansprüche 6 bis 10, dadurch gekennzeichnet, daß man zu der wässrigen Mehlsuspension ein Plastifizierungsmittel hinzufügt ausgewählt unter Glyzerol, den Polyalkoholen, den Glyzeriden, den Phospholipiden, den Lipiden einzeln oder in Mischung mit einer Konzentration zwischen 1 und 30 % bezogen auf das Mehl.

12. Verfahren nach Anspruch 11, dadurch gekennzeichnet, daß das Plastifizierungsmittel Glyzerol ist.

13. Verfahren nach Anspruch 11 oder 12, dadurch gekennzeichnet, daß das Plastifizierungsmittel ein eßbares Öl ist.

14. Verfahren nach einem der Ansprüche 6 bis 13, dadurch gekennzeichnet, daß die wässrige Suspension des Mehls und der Plastifizierungsmittel durch Erhitzung im Wasserbad geliert ist.

15. Verfahren nach Anspruch 14, dadurch gekennzeichnet, daß die Suspension während 6 bis 15 Minuten auf eine Temperatur von 90 bis 100 °C erhitzt ist.

16. Verfahren nach einem der Ansprüche 6 bis 15, dadurch gekennzeichnet, daß die gelierte Suspension homogenisiert wird, dann in einer gleichmäßigen Schicht auf einen nicht klebenden Träger aufgetragen wird und schließlich bis zum Erhalt einer Folie getrocknet wird.